Europäisches Patentamt

European Patent Office

Office européen des brevets

(11) Publication number: **0 259 078**
**A1**

# EUROPEAN PATENT APPLICATION

(21) Application number: 87307448.8

(22) Date of filing: 24.08.87

(51) Int. Cl.4: **A61K 33/18** ,
//(A61K33/18,31:765)

(30) Priority: 30.08.86 GB 8621008

(43) Date of publication of application:
09.03.88 Bulletin 88/10

(84) Designated Contracting States:
AT BE CH DE ES FR GB IT LI NL SE

(71) Applicant: Smith and Nephew Associated
Companies p.l.c.
2, Temple Place Victoria Embankment
London WC2R 3BP(GB)

(72) Inventor: Edwards, Linda Mary
8 Walden Road Littlebury
Saffron Walden Essex(GB)

(74) Representative: Cole, William Gwyn
Corporate Patents Department Smith and
Nephew Research Limited Gilston Park
Harlow Essex CM20 2RQ(GB)

(54) Pharmaceutical composition.

(57) A sterile pharmaceutical composition which is suitable for topical application to the skin comprises from 20 to 60% by weight of iodophor in a water dispersible pharmaceutically acceptable carrier. In a preferred composition the iodophor is a polyvinylpyrrolidone-iodine complex and the water dispersible carrier a mixture of high and low molecular weight polyethylene glycols.

EP 0 259 078 A1

## PHARMACEUTICAL COMPOSITION

The present invention relates to a sterile pharmaceutical composition suitable for topical application to the skin which composition contains an iodophor in a water dispersible pharmaceutically acceptable carrier.

Many antibacterial agents when used topically on the skin as a surgical scrub or in the treatment of lesions such as burns often fail to realise their expected antibacterial efficiency. This may be due to one or more of the following reasons. Firstly the components of the carrier for the antibacterial agent may reduce the effectiveness of the antibacterial agent. Secondly the presence of serum or other proteins and fatty material may deactivate the antibacterial agent before it is able to act effectively. Iodophenylacetic are particularly susceptible to the latter form of deactivation. It would be advantageous therefore to provide a composition of an iodophor which did not suffer from these disadvantages. Surprisingly I have found that by including the iodophor in the percentage range claimed in a water dispersible carrier, a composition is obtained which may be applied topically to the skin and wounds and does not show the loss of bactericidal activity shown by other compositions containing an equivalent amount of iodine.

Accordingly the present invention provides a sterile pharmaceutical composition suitable for topical application to the skin which composition contains 20 to 60% by weight of iodophor in a water dispersible pharmaceutically acceptable carrier.

Favourably the compositions of the present invention are in the form of an ointment. Ointments are semisolid preparations for topical application to the body which usually soften, but not necessarily melt, when applied to the skin. A preferred ointment is a so-called water-soluble ointment. Water-soluble or water dispersible ointment bases usually contain polyethylene glycol polymers or derivatives thereof which are available as Carbowaxes (Trade mark). Suitable derivatives include for example the monostearate.

Suitably the iodophor will be present in an amount of 20 to 60% by weight of the composition, more suitably will comprise from 25 to 40% by weight of the composition and preferably will comprise from 27 to 35% by weight, for example 28%, 30%, 32% and 34%.

Aptly the iodophor may be any one or mixture of those iodophors which are known in the art to show antibacterial properies including polyvinylpyrrolidone-iodine, nonylphenoxypoly(ethylenoxy)ethanol-iodine and undecoliniumchloride-iodine. A preferred iodophor is a polyvinylpyrrolidone-iodine complex. Polyvinylpyrrolidone-iodine complexes normally contain from 9 to 12% by weight of available iodine and most commercial materials contain 10% by weight available iodine.

Aptly the water dispersible carrier is polyalkylene glycol either as a single component or a mixture of two or more polyalkylene glycols differing in molecular weight and/or the nature of the alkyl group. Suitably the alkyl group may contain from 1 to 4 carbon atoms. Thus a suitable carrier comprises from 80 to 40% by weight of polyethylene glycol and a particularly suitable carrier comprises a polyethylene glycol of low molecular weight for example 200 to 1600 mixed with a polyethylene glycol of high molecular weight, for example 2600 to 6000, whereby the low molecular weight glycol comprises from 40 to 60% by weight of the composition and the high molecular weight glycol comprises from 0 to 20% by weight of the composition.

A preferred aspect of the present invention therefore comprises a sterile pharmaceutical composition suitable for topical application to the skin which composition contains 20 to 60% by weight of polyvinylpyrrolidone-iodine, 80 to 40% by weight of a polyethylene glycol having a molecular weight between 200 and 1600 and 0 to 20% by weight of a polyethylene glycol having a molecular weight between 2600 and 6000.

The compositions of this invention are sterile or are capable of being sterilised. The compositions may be prepared by simply mixing the ingredients together usually at elevated temperature so that the water dispersible components are liquid and allowing to cool. The temperature employed may be such that if the mixture is maintained at this temperature for a period of time, the mixture may be prepared in a sterile form. On cooling the sterile mixture may be filled into presterilised tubes or pots or other packages used for dispensing viscous compositions. Alternatively the composition may be packaged into an appropriate container and the entire container and contents sterilised for example by irradiation. Surprisingly the compositions of the present invention are stable to sterilisation.

The compositions of the present invention may be used for the treatment or prevention of infection in wounds caused by cuts and abrasions, following surgical procedures, burns, decubitus and stasis ulcers. They may also be used in the treatment of mycotic and bacterial skin infections and may also be used prophylactically for pre-operative degerming of the skin.

In use the compositions of the present invention may be applied topically to the skin or wound in the normal manner, for example by spreading and smoothing over the affected area using a knife, a spatula sterile or a sterile gloved hand. One of the advantages of the compositions of the present invention is that they may be washed from the skin with water so that the composition may be removed easily and new treatment initiated. The compositions may be applied liberally and may be covered by a dressing such as multiple layers of gauze or cotton wool or a bandage. The compositions may be used as often as required at the discretion of the physician. However in the treatment of wounds such as burns treatment may be renewed each six to eight hours while in other conditions treatment may be daily. In degerming of the skin the composition is effective up to 6 hours. The frequency of treatment may vary between once every four hours and once every twenty-four hours. Treatment using compositions of the present invention will usually continue for up to 4 days where appropriate, though for ulcers the treatment period may be much longer.

In the treatment of burns, ulcers and infected wounds the composition may be applied as a layer from 3 to 5mm thick providing an effective dose of free iodine of between 5 and 25mg/sq.cm, this when using an iodophor containing 10% free iodine.

Alternatively the compositions of this invention may be used in combination with conventional dressings which are used to provide topical application of medicaments, and in particular antibacterial agents, to wounds such as tulle gras, foam or fabric pads or gels. Such dressings are preferably sterile and are packaged in bacteria-proof, waterproof packages before use.

In another aspect the present invention comprises a method of treating wounds which comprises topically applying to the affected area an effective amount of a sterile pharmaceutical composition which composition contains 20 to 60% by weight of iodophor in a water dispersible pharmaceutically acceptable carrier.

In a preferred aspect the present invention comprises a method of treating wounds which method comprises applying topically to the affected area an effective amount of a sterile pharmaceutical composition which composition contains 20 to 60% by weight of polyvinylpyrrolidone-iodine, 80 to 40% by weight of a polyethylene glycol of molecular weight between 200 and 1600 and 0 to 20% by weight of a polyethylene glycol of molecular weight between 2600 to 6000.

Example 1

A pharmaceutical composition was prepared having the following composition:
Polyvinylpyrrolidone-iodine      30g
Polyethylene glycol (M.W.400)      55g
Polyethylene glycol (M.W.4000)      15g
The composition was prepared by mixing together the glycol components and then adding the polyvinylpyrrolidone-iodine until a homogeneous mixture was achieved. The composition may be sterilised by gamma irradiation at 2.5 Mrad and filled into presterilised plastic pots or tubes.

Example 2

A pharmaceutical composition was prepared having the following composition:
Polyvinylpyrrolidone-iodine      40g
Polyethylene glycol (M.W.600)      60g
The composition was prepared and may be packaged as described in Example 1.

Example 3

A pharmaceutical composition was prepared having the following composition:
Polyvinylpyrrolidone-iodine      25g
Polyethylene glycol (M.W.400)      55g
Polyethylene glycol (M.W.4000)      15g
The composition was prepared and may be packaged as described in Example 1.

### Example 4

A sterile pharmaceutical composition was prepared having the following composition:

Undecolinium chloride-iodine    301% (w/w)
Polyethylene glycol (M.W.400)    56%
Polyethylene glycol (M.W.4000)    14%

The composition was prepared by mixing together with warming the undecolinium chloride-iodine with the low molecular weight polyethylene glycol until a solution is achieved and then adding the high molecular weight polyethylene glycol with stirring. Stirring is continued until the mixture is homogeneous. The mixture is allowed to cool and is sterilised by irradiation with gamma rays at 2.5 Mrad.

### Example 5

A sterile pharmaceutical composition was prepared having the following composition:

Nonylphenoxypoly(ethyleneoxy)-ethanol iodine    40%(w/w)
Polyethylene glycol (M.W.400)    50%
Polyethylene glycol (M.W.4000)    10%

The composition was prepared by mixing together, with warming, all three components. Stirring was continued until the mixture was homogeneous and until the mixture cooled to ambient room temperature. The composition is sterilised by irradiation.

The sterile composition may be filled into presterilised plastic pots and tubes under aseptic conditions.

### Demonstration of Effectiveness

A weighed sample of ointment containing polyvinylpyrrolidone-iodine was placed in the bottom of a vial which was secured in a clamp and a stirrer was then lowered into the vial. Serum (1.0ml) was added to the vial and the stirrer was activated (200rpm). At specified time intervals 25$\mu$l aliquots of the serum were removed and placed into 1.0ml of 2% aqueous potassium iodide solution and mixed.

These solutions were then scanned on a UV-vis spectrophotometer at a wave length between 400 and 300nm. The absorbance of the peak at 355nm was measured and, after correction for a serum blank, was used to calculate the polyvinylpyrrolidone-iodine content of the aliquot from a calibration graph prepared from standard solutions of polyvinylpyrrolidone-iodine in potassium iodide solution. These values were used to calculate the polyvinylpyrrolidone-iodine solution present in the serum layer after correction for volume losses.

Two samples of ointment were used, namely an ointment 1 containing 10% polyvinylpyrrolidone-iodine, 70% polyethylene glycol (M.W. 400) and 20% polyethylene glycol (M.W. 4000) and an ointment 2 as described in Example 1. The weight of the ointment 1 taken was 125mg. The weight of ointment 2 taken was 43mg so that the same amount of polyvinylpyrrolidone-iodine and hence iodine was present in each sample. The amount of available iodine was measured for each sample using the procedure given above at various times after contacting with serum.

The results are shown in Table 1.

| Ointment serum contact time (min) | Available iodine level (µg/ml) | |
| --- | --- | --- |
| | Ointment 1 | Ointment 2 |
| 0 | 0.0 | 0.0 |
| 1 | 3.2 | 5.4 |
| 3 | 12.2 | 15.9 |
| 5 | 11.2 | 20.2 |
| 10 | 6.7 | 18.0 |
| 15 | 4.6 | 13.7 |
| 30 | 1.4 | 5.6 |

Since the two ointments contain the same amount of iodine it would be expected that the available iodine from the two ointments would be the same. Surprisingly it is observed that the available iodine from ointment 2 is consistently higher than that from ointment 1 and is higher for a longer period.

The comparison of available iodine measured analytically with antimicrobial activity was measured by making samples containing 250µl of 50mg/ml of polyvinylpyrrolidone-iodine and adding to each 1ml of serum. Each sample was challenged by an inoculum at various times after mixing with serum and a log reduction of inoculum was measured at 2 and 5 minutes after challenge. These results were compared with the amount of free iodine found by analytical means. The challenge was by $10^7$ organisms/ml of Staphylococcus aureus NCTC 10788.

The results are shown in Table 2.

| Polyvinylpyrrolidone-iodine and serum contact time before addition of inoculum (mins) | Available iodine measured analytically (µg/ml) | Log reduction of inoculum | |
| --- | --- | --- | --- |
| | | 2min post challenge | 5min post challenge |
| 0 | 0 | – | – |
| 5 | 7.2 | 3.59 | 4.08 |
| 10 | 4.3 | 1.76 | 2.96 |
| 15 | 3.5 | 1.08 | 1.22 |
| 30 | 1.6 | 0.63 | 0.69 |

These results when read with Table 1 shows that the ointments of the present invention are unexpectedly more antimicrobially effective in the presence of serum.

**Claims**

1. A sterile pharmaceutical composition suitable for topical application to the skin which composition contains 20 to 60% by weight of iodophor in a water dispersible pharmaceutically acceptable carrier.

2. A composition according to claim 1 in which the composition is in the form of an ointment.

3. A composition according to claim 2 in which the composition contains from 25 to 40% by weight of iodophor.

4. A composition according to claim 2 in which the iodophor is a polyvinylpyrrolidone-iodine complex.

5. A composition according to claim 1 in which the water dispersible carrier is polyalkylene glycol.

6. A composition according to claim 5 in which the polyalkylene glycol is polyethylene glycol.

7. A composition according to claim 6 in which the polyethylene glycol comprises a mixture of from 40 to 60% by weight of a polyethylene glycol comprises a mixture of from 40 to 60% by weight of a polyethylene glycol with a molecular weight of from 200 to 1600, and 0 to 20% by weight of a polyethylene glycol with a molecular weight of from 2000 to 6000.

8. A sterile wound dressing comprising a tulle gras when containing a composition comprising 20 to 60% by weight of iodophor in a water dispersible carrier.

9. A pharmaceutical composition which is capable of being sterilised and which contains 20 to 60% by weight of iodophor in a water dispersible pharmaceutically acceptable carrier.

# EUROPEAN SEARCH REPORT

Application Number

EP 87 30 7448

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 124 774 (INTERMEDICAT GmbH) <br> * Page 9; claims 1-6 * <br> --- | 1-9 | A 61 K 33/18 // <br> (A 61 K 33/18 <br> A 61 K 31:765) |
| X | US-A-4 401 651 (RICHARD A. KNUTSON) <br> * Column 2, lines 7-54; column 6, lines 10-68 * <br> --- | 1-9 | |
| X | FR-A-2 138 295 (MUNDIPHARMA AF) <br> * Page 30, line 1 - page 31, line 15; claims 1-6 * <br> ----- | 1-9 | |

TECHNICAL FIELDS SEARCHED (Int. Cl.4)

A 61 K

The present search report has been drawn up for all claims

| Place of search | Date of completion of the search | Examiner |
|---|---|---|
| THE HAGUE | 04-12-1987 | BRINKMANN C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document